(19) Europäisches Patentamt
European Patent Office
Office européen des brevets

(11) Publication number: **0 495 981 A1**

(12) **EUROPEAN PATENT APPLICATION published in accordance with Art. 158(3) EPC**

(21) Application number: **90912465.3**

(22) Date of filing: **28.08.90**

(86) International application number: **PCT/JP90/01088**

(87) International publication number: **WO 92/03416 (05.03.92 92/06)**

(43) Date of publication of application: **29.07.92 Bulletin 92/31**

(84) Designated Contracting States: **DE FR GB**

(51) Int. Cl.5: **C07D 207/273**, A01N 43/36

(71) Applicant: **MITSUI TOATSU CHEMICALS, Inc.**
**2-5 Kasumigaseki 3-chome**
**Chiyoda-Ku Tokyo 100(JP)**

(72) Inventor: **ODA, Kengo**
**522, Fujimi shataku, 2225, Togo**
**Mobara-shi, Chiba 297(JP)**
Inventor: **MORIYASU, Koichi**
**38, Fujimi apaato, 2225-1, Togo**
**Mobara-shi, Chiba 297(JP)**
Inventor: **ARAI, Kiyoshi**
**25, Miyanodaidaini apaato, 2142, Togo**
**Mobara-shi, Chiba 297(JP)**
Inventor: **NISHIDA, Makoto**
**35, Miyanodaidaini apaato, 2142, Togo**
**Mobara-shi, Chiba 297(JP)**
Inventor: **OYAMADA, Masami**
**2787, Honnou**
**Mobara-shi, Chiba 299-41(JP)**
Inventor: **FUJIWARA, Akie**
**241, Hachimanmae-shataku, 138-1, Machibo**
**Mobara-shi, Chiba 297(JP)**

(74) Representative: **Zumstein, Fritz, Dr. et al**
**Zumstein & Klingseisen Patentanwälte**
**Bräuhausstrasse 4**
**W-8000 München 2(DE)**

(54) **4-ETHYL-1-(3-TRIFLUOROMETHYLPHENYL)-2-PYRROLIDINONE DERIVATIVES AND HERBICIDES CONTAINING THE SAME AS ACTIVE INGREDIENT.**

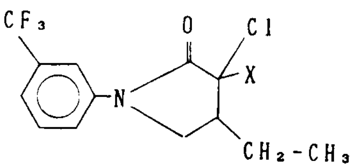

( I )

(57) 4-Ethyl-1-(3-trifluoromethylphenyl)-2-pyrrolidinone derivatives represented by general formula (I), which exhibit such an excellent selective herbicidal activity that they kill detrimental weeds only in small amounts of application without damaging useful crops in both lowland and upland farmings, wherein X represents chlorine or hydrogen.

EP 0 495 981 A1

## TECHNICAL FIELD

The present invention relates to novel 4-ethyl-1-(3-trifluoromethylphenyl)-2-pyrrolidinone derivatives and herbicidal compositions containing same as active ingredients.

## BACKGROUND ART

Possession of herbicidal activities by certain types of 2-pyrrolidinone derivatives has already been disclosed, for example, in Japanese Patent Application Laid-Open No. 89666/1977, Japanese Patent Application Laid-Open No. 154558/1983, Japanese Patent Application Laid-Open No. 58960/1985, etc. Further, preparation processes of 2-pyrrolidinone derivatives are disclosed in U.S. Patent No. 4,132,713.

3-Chloro-4-(chloromethyl)-1-(3-trifluoromethylphenyl)-2-pyrrolidinone(generally called "fluorochloridone") which is a typical example of the compounds disclosed in Japanese Patent Application Laid-Open No. 89666/1977 and Japanese Patent Application Laid-Open No. 154558/1983 has been available commercially.

The compounds disclosed in the above Japanese Patent Application Laid-Open No. 89666/1977 and those disclosed in Japanese Patent Application Laid-Open No. 154558/1983 require relatively high application rates when employed as herbicides, and the use of these compounds in paddy fields at an application rate effective against certain harmful weeds results in serious injury to rice plants (*Oryza sativa*) therein.

## DISCLOSURE OF THE INVENTION

Objects of the present invention are therefore to provide compounds having selective herbicidal activities such that harmful weeds can be killed even at a low application rate without giving damages to economic crops in paddy fields and upland fields and also to provide herbicidal compositions containing these compounds as active ingredients.

The present inventors have proceeded with a further investigation on 2-pyrrolidinone derivatives with a view toward obtaining herbicides which, compared to the conventional herbicides, have excellent effects even at lower application rates and do not cause crop injury. As a results, it has been found that novel 4-ethyl-1-(3-trifluoromethylphenyl)-2-pyrrolidinone derivatives having an ethyl group at the 5-position of the pyrrolidinone ring have characteristic features of extremely good herbicidal effects and moreover no injury to economic crops.

The present invention provides 4-ethyl-1-(3-trifluoromethylphenyl)-2-pyrrolidinone derivatives represented by the following formula (I):

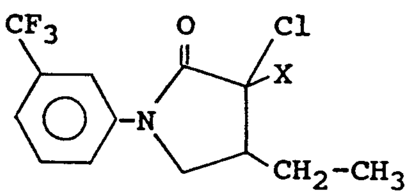

(I)

wherein X represents a chlorine or hydrogen atom and also herbicidal compositions containing these compounds as active ingredients.

The compounds disclosed in Japanese Patent Application Laid-Open No. 89666/1977 or Japanese Patent Application Laid-Open No. 154558/1983 require a relatively high application rate when applied in a field to control principal weeds. They therefore give serious injury to crops especially in paddy fields and upland fields, so that their use is extremely limited. The compounds according to the present invention can be applied at lower rates to upland crops and moreover, have high safety for rice plants (*Oryza sativa*) in paddy fields. As a result, the applicable range has become extremely wide.

The compounds of the present invention have a similar structure to the above-described compounds of the prior art except that the chloromethyl group at the 4-position of the pyrrolidinone ring has been changed to an ethyl group. As a result of this conversion, however, not only their activities as herbicides have been enhanced but also the difference in selectivity between the crop and weeds in a field has been widened, whereby their applications in paddy fields and upland fields have hence become easier.

## BEST MODE FOR CARRYING OUT THE INVENTION

The herbicidal compositions containing one or more compounds of this invention as active ingredients have, as their characteristic activities, herbicidal activity against most of the harmful weeds which cause problems in paddy fields or upland fields, for example, gramineous weeds such as barnyardgrass (*Echinochloa*), cyperaceous weeds such as *Cyperus microiria* and bulrush (*Scirpus juncoides*), and perennial broadleaf weeds such as *Sagittaria pygmaea* in paddy fields; and broadleaf weeds such as amaranth (*Amaranthus viridis*), henbit (*Lamium amplexicaule*), chickweed (*Stellaria media*) and common lambsquarters, and gramineous weeds such as crabgrass (*Digitaria adscendens*), wild oat (*Avena fatua*), foxtail (*Setaria viridis*), annual bluegrass (*Poa annua L.*) and foxtail water (*Alopecurus aequalis*) in upland fields. Nevertheless, they cause no injury to economic crops such as rice (*Oryza sativa*), wheat (*Triticum*), corn (*Zea maize*), cotton (*Gossypium indicum*), soybean (*Glycine max*), etc.

Further, the herbicidal compositions of the compounds according to the present invention are effective when applied by any application methods such as submerged soil application, soil application, soil incorporation or foliar application.

In soil application and soil incorporation in particular, no variations are observed in effects and injury depending on physical factors such as soil water content and soil quality.

The 4-ethyl-1-(3-trifluoromethylphenyl)-2-pyrrolidinone derivatives according to the present invention are novel compounds and can be prepared by the following process.

They can each be prepared easily in a single step by subjecting an amide derivative represented by the formula (II) to the following reductive cyclizing reaction:

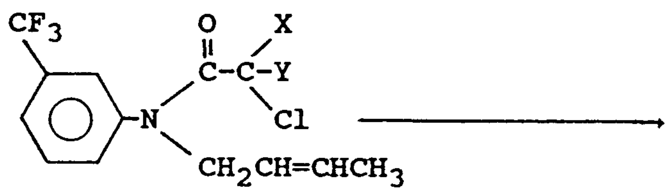

(I)

(II)

wherein X has the same meaning as defined above and Y represents a halogen atom.

Exemplary cyclizing agents effective for the above cyclizing reaction include tributyltin hydride. The reaction is generally conducted in an aromatic solvent, such as benzene, toluene or xylene. When X is a chlorine atom in the formula (II), the use of tributyltin hydride in an equimolar amount relative to the compound of the formula (II) results in the dichloro derivative [i.e., the compound of the formula (I) in which X is a chlorine atom] while its use in a twofold molar amount leads to the monochloro derivative [i.e., the compound of the formula (I) in which X is a hydrogen atom]. Further, when X is a hydrogen atom, the monochloro derivative is obtained by the use of tributyltin hydride in an equimolar amount. The reaction temperature is preferably 50-140 °C. The reaction proceeds by the addition of a catalytic amount of a radical generating agent, such as $\alpha,\alpha$-azobisisobutyronitrile or benzoyl peroxide, to the reaction mixture. Radiation is also effective.

The amide derivatives represented by the formula (II) can each be prepared by reacting an amine of the formula (III) and a carboxylic acid derivative:

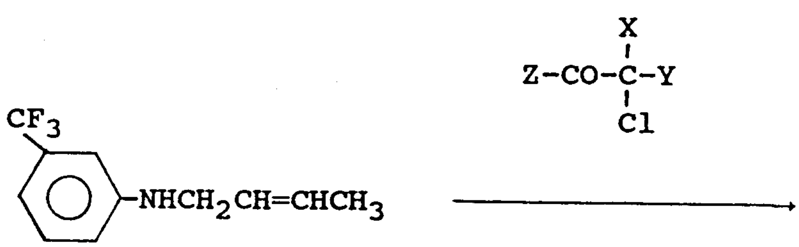

(II)

(III)

wherein X and Y have the same meanings as defined above and Z represents a halogen atom.

The reaction is conducted in the absence of a solvent or in an inert solvent. Illustrative inert solvents include aromatic hydrocarbons such as benzene, toluene, xylene, chlorobenzene and dichlorobenzene, halogenated aliphatic hydrocarbons such as dicloromethane, chloroform and carbon tetrachloride, ethers

such as diethyl ether, tetrahydrofuran and dioxane, and esters such as ethyl acetate and butyl acetate. The reaction proceeds at any desired temperature. It is possible to carry out the reaction in the presence of a base such as triethylamine, pyridine, N,N-dimethylaniline, sodium hydride, potassium hydride, sodium carbonate, potassium carbonate and sodium bicarbonate.

The amines of the formula (III) can be prepared by the process described in U.S. Patent No. 4,132,713 or the like.

To use the compounds of the formula (I) according to this invention, which can be obtained as described above, as herbicides, they are generally mixed with an inert liquid or solid carrier and then formed into a commonly-used formulation such as powder, granules, wettable powder, emulsion and flowable formulation. One or more auxiliary agents can also be added if necessary for formulation purposes.

Any carrier can be used as long as it is usable in conventional agricultural or horticultural chemicals, no matter whether it is solid or liquid. No particular limitation is therefore imposed on the carrier. Exemplary solid carriers include mineral powders such as clay, talc, bentonite, calcium carbonate, diatomaceous earth and white carbon; vegetable powders such as soybean flour and starch; high molecular compounds such as petroleum resins, polyvinyl chloride alcohol and polyalkylene glycols; urea; and waxes. Illustrative liquid carriers include various organic solvents such as xylene, methylnaphthalene and alkylbenzenes; and water.

As auxiliary agents, surfactants, binders, stabilizers and the like which are generally used in agricultural or horticultural chemicals can be used either singly or in combination. In some instances, industrial bactericides, antiseptics and the like can also be incorporated for the control of bacteria and fungi.

As exemplary surfactants, non-ionic, anionic, cationic and amphoteric surfactants can be used either singly or in combination. Those obtained by adding ethylene oxide or propylene oxide to alkyl phenols, higher alcohols, alkylnaphthols, higher fatty acids, fatty acid esters and the like can be used as preferred non-ionic surfactants. Exemplary anionic surfactants include the alkylsulfonate salts, alkyl sulfate ester salts, phosphate ester salts and the like of alkylphenols, alkylnaphthols, higher alcohols, higher fatty acids, fatty acid esters and the like. Lignine sulfonate salts and the like are also preferred.

The content of each compound of the formula (I) in the herbicide according to the invention varies depending on the formulation and end use. In general, it is 0.05-20 wt.% in a powder, 1-50 wt.% in a wettable powder, 0.05-10 wt.% in a granule, 1-50 wt.% in an emulsion, 1-50 wt.% in a flowable formulation and 1-50 wt.% in a dry flowable formulation. Preferably, it is 0.5-5 wt.% in a powder, 10-40 wt.% in a wettable powder, 0.5-5 wt.% in a granule, 10-20 wt.% in an emulsion, 20-30 wt.% in a flowable formulation and 20-40 wt.% in a dry flowable formulation.

The herbicides of the invention may be used in combination with one or more of other herbicides, bactericides, insecticides, plant growth regulators, fertilizers and soil improving agents. In some instances, certain synergistic effects may be expected from such combined use.

The process for the synthesis of the compounds of the invention will be described by the following examples.

Example 1

Synthesis of 4-ethyl-3,3-dichloro-1-(3-trifluoromethylphenyl)-2-pyrrolidinone (Compound No. 1)

N-(2-Butenyl)-N-(3-trifluoromethylphenyl)-2,2,2-trichloroacetamide (1.5 g) was added to 15 mℓ of benzene, followed by the addition of 1.2 g of tributyltin hydride and an extremely small amount of $\alpha,\alpha$-azobisisobutyronitrile (AIBN) under stirring at the refluxing temperature. After the reaction mixture was continuously stirred for 10 minutes, 40 mℓ of saturated saline were added, followed by extraction with toluene. After the extract was dried over anhydrous magnesium sulfate, the extract was concentrated in an evaporator and then purified by chromatography on a silica gel column (hexane/ethyl acetate: 20/1, v/v) so that 1.1 g of the title compound were obtained.

Example 2

Synthesis of 4-ethyl-3-chloro-1-(3-trifluoromethylphenyl)-2-pyrrolidinone (Compound Nos. 2 & 3)

N-(2-Butenyl)-N-(3-trifluoromethylphenyl)-2,2,2-trichloroacetamide (1.5 g) was added to 20 mℓ of toluene, followed by the addition of 2.4 g of tributyltin hydride and an extremely small amount of $\alpha,\alpha$-azobisisobutyronitrile (AIBN) under stirring at 70 °C. After the reaction mixture was continuously stirred for 1 hour, 80 mℓ of 20% hydrochloric acid were added, followed by extraction with toluene. After the extract was dried over anhydrous sodium sulfate, the extract was concentrated in an evaporator and then subjected to

silica gel chromatography (hexane/ethyl acetate: 15/1, v/v) so that the 3,4-trans isomer (0.72 g) and the 3,4-cis isomer (0.25 g) were obtained.

The thus-obtained compounds of the formula (I) according to the present invention and their physical properties are shown in Table 1.

Table 1

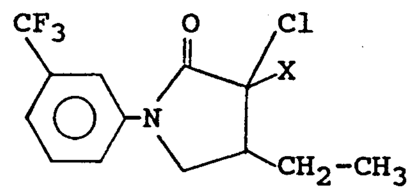

(I)

| Comp'd No. | Substituent in formula (I) X | Physical properties |
|---|---|---|
| 1 | Cl | NMR ($CDCl_3$) δ ppm: 1.15(3H,t,J=8Hz), 1.60-2.24(2H,m), 2.60-3.00(1H,m), 3.57(1H,dd,J=8Hz,J=9Hz), 3.91(1H,dd,J=8Hz,J=7Hz), 7.45-7.65(2H,m), 7.80-8.04(2H,m). IR ν neat $cm^{-1}$: 1700. m.p.: 67.0-68.0°C |
| 2 | H 3,4-trans | NMR ($CDCl_3$) δ ppm: 1.12(3H,t,J=8Hz), 1.50-1.90(2H,m), 2.40-2.80(1H,m), 3.56(1H,dd,J=8Hz,J=8Hz), 4.03(1H,dd,J=8Hz,J=8Hz), 4.26(1H,d,J=9Hz), 7.30-7.70(2H,m), 7.80-8.04(2H,m). IR ν neat $cm^{-1}$: 1690. |
| 3 | H 3,4-cis | NMR ($CDCl_3$) δ ppm: 1.07(3H,t,J=8Hz), 1.40-1.80(2H,m), 2.40-2.80(1H,m), 3.70-4.00(2H,m), 4.50(1H,d,J=6Hz) 7.30-7.70(2H,m), 7.80-8.04(2H,m). IR ν neat $cm^{-1}$: 1690. |

Further, some synthesis examples of intermediates for the preparation of the compounds of formula (I) according to the present invention will next be described as referential examples.

Referential Example 1

Synthesis of N-(2-butenyl)-N-(3-trifluoromethylphenyl)-2,2,2-trichloroacetamide

In 40 mℓ of toluene, 2.7 g of trichloroacetyl chloride were gradually added dropwise at room temperature under stirring to 2.2 g of N-(2-butenyl)-N-(3-trifluoromethylphenyl)amine. After the reaction mixture was stirred for further 20 minutes, the precipitated insoluble matter was filtered off and 50 mℓ of toluene were added. The resulting toluene solution was washed twice with a saturated aqueous solution of sodium bicarbonate and twice with saturated saline. The toluene solution was dried over anhydrous sodium sulfate, concentrated in an evaporator and then subjected to chromatography on a silica gel column (hexane/ethyl acetate: 25/1, v/v), whereby the title compound was stoichiometrically obtained.
IR $\nu$ neat $cm^{-1}$: 1685.
$N_D$23.0 °C: 1.4714.

Referential Example 2

Synthesis of N-(2-butenyl)-N-(3-trifluoromethylphenyl)amine

In 30 mℓ of dimethylformamide, 1.4 g of anhydrous potassium carbonate and 1.0 g of 1-chloro-2-butene were added to 1.6 g of 3-aminobenzotrifluoride, followed by stirring for 2 hours at 70-90 °C. After potassium carbonate was filtered off, 100 mℓ of saturated saline were added and the resulting mixture was extracted with benzene. The extract was dried over anhydrous sodium sulfate, concentrated and then subjected to chromatography on a silica gel column (hexane/ethyl acetate: 35/1, v/v), whereby the title compound was obtained.
IR $\nu$ neat $cm^{-1}$: 3400.
$N_D$22.8 °C: 1.4903.
Yield: 65.5%.

Formulation examples and herbicidal activity tests of certain herbicides according to the present invention will next be described.

Formulation Example 1 (Wettable powder)

A wettable powder was obtained by thoroughly grinding and mixing 20 parts by weight of Compound No. 1 of the present invention, 2 parts by weight of "Neopelex" (trade mark, product of Kao Corporation; sodium dodecyl benzene sulfonate), 2 parts by weight of "Neugen EA" (trade name, product of Daiichi Kogyo Seiyaku Industries, Ltd.; polyoxyethylene nonylphenyl ether), 5 parts by weight of white carbon and 71 parts by weight of diatomaceous earth.

Formulation Example 2 (Powder)

A powder was obtained by thoroughly grinding and mixing 1 part by weight of Compound No. 1 of the present invention, 0.5 part by weight of "Emulgen 910" (trade name, product of Kao Corporation; polyoxyethylene nonylphenyl ether) and 98.5 parts by weight of kaolin clay.

Formulation Example 3 (Granule)

One part by weight of Compound No. 2 of the present invention, 2 parts by weight of "Neopelex" (trade mark; described above), 2 parts by weight of "Sun Ekisu P252" (trade name; product of Sanyo-Kokusaku Pulp Co., Ltd.; sodium lignine sulfonate), 72 parts by weight of bentonite and 23 parts by weight of talc were thoroughly mixed. A suitable amount of water was added to the resultant mixture to wet the same, followed by extrusion of the mass through a small injection molding machine into pellets. After the pellets were dried at 30-60 °C in air and then crushed into granules, the granules were classified by a sifting machine to collect granules of 0.3-2 mm, whereby a granular formulation was obtained.

Formulation Example 4 (Emulsion)

An emulsion was obtained by mixing 10 parts by weight of Compound No. 2 of the present invention, 10 parts by weight of "Sorpole 800A" (trade name, product of Toho Chemical Industries Co., Ltd.; a nonionic/anionic surfactant mixture) and 80 parts by weight of o-xylene.

Formulation Example 5: (Flowable formulation)

A flowable formulation was obtained by wet grinding and mixing 30 parts by weight of Compound No. 3 of the present invention and a solution of 10 parts by weight of "Sun Ekisu P252" (trade name, described above) in 50 parts by weight of water and then adding and mixing a solution of 0.2 part by weight of "Kelzan S" (trade name, product of Kelco Corp.; xanthan gum) in 9.6 parts by weight of water and 0.2 part by weight of "Deltop" (trade mark, product of Takeda Chemical Industries, Ltd.; organic iodine fungicide).

Test 1 Treatment of Soil under Submerged Condition (Pre-emergence Treatment)

1/5000-are Wagner pots were filled with soil. Seeds or tubers of *Echinochloa crusgalli*, bulrush (*Scirpus juncoides*), monochoria (*Monochoria vaginalis*), *Sagittaria pygmaea* and water nutgrass (*Cyperus serotinus*) were seeded or planted under submerged condition. Two pairs of rice (*Oryza sativa*) seedlings (2-3 leaf stage), which had been reared in advance, were transplanted to each pot and were allowed to grow in a green house. Each pair consisted of two rice seedlings. One day later (before emergence of weeds), each pot was treated with a granule which had been prepared by processing a predetermined amount of the test compound in accordance with a similar method to the method described in Formulation Example 3. The state of emergence of weeds and the state of injury of rice were observed 30 days later.

The results are summarized in Table 2.

In the table, the degree of damages of each test plant and the degree of injury to rice were determined by comparing the state of growth of the plant with that of the plant in untreated pots in terms of air-dry weight and are shown in accordance with the following standard:

| Rank | Growth rate (%) expressed in terms of the percentage of air-dry weight relative to that of untreated group |
|---|---|
| 5 | 0 - 5 (Death) |
| 4 | 6 - 10 (Severe damages) |
| 3 | 11 - 40 (Medium damages) |
| 2 | 41 - 70 (Small damages) |
| 1 | 71 - 90 (Slight damages) |
| 0 | 91 -100 (No damages) |

Comparative Compounds A and B represent the following compounds, respectively (this will also apply to Test 2 and Test 3):

A: 1-(3-trifluoromethylphenyl)-3-chloro-4-chloromethyl-2-pyrrolidinone.

B: 1-methyl-3-phenyl-5-(3-trifluoromethylphenyl)-pyridin-4(1H)-one.

In the present test, the compounds according to the present invention exhibited, compared with Comparative Compounds A and B, higher herbicidal effects against the tested paddy field weeds and excellent safety to rice (*Oryza sativa*).

Table 2 Submerged Soil Treatment Test

| Compound No. | Application rate, kg/ha | *Echinochloa crusgalli* | Monochoria (*Monochoria vaginalis*) | Bulrush (*Scirpus juncoides*) | *Sagittaria pygmaea* | Water nutgrass (*Cyperus serotinus*) | Rice (*Oryza sativa*) |
|---|---|---|---|---|---|---|---|
| 1 | 0.1 | 4 | 4 | 3 | 3 | 3 | 0 |
| | 0.2 | 5 | 5 | 4 | 3 | 4 | 0 |
| | 0.4 | 5 | 5 | 5 | 5 | 5 | 0 |
| 2 | 0.1 | 4 | 5 | 3 | 4 | 4 | 0 |
| | 0.2 | 5 | 5 | 5 | 4 | 5 | 0 |
| | 0.4 | 5 | 5 | 5 | 5 | 5 | 0 |
| 3 | 0.1 | 2 | 2 | 2 | 2 | 2 | 0 |
| | 0.2 | 4 | 3 | 3 | 3 | 3 | 0 |
| | 0.4 | 5 | 4 | 4 | 4 | 4 | 0 |
| A | 0.1 | 2 | 3 | 2 | 1 | 2 | 2 |
| | 0.2 | 3 | 4 | 3 | 3 | 3 | 3 |
| | 0.4 | 5 | 5 | 4 | 4 | 5 | 5 |
| B | 0.1 | 3 | 3 | 3 | 3 | 2 | 2 |
| | 0.2 | 4 | 4 | 4 | 4 | 3 | 4 |
| | 0.4 | 5 | 5 | 5 | 4 | 4 | 5 |

Comparative Compounds:
A: 1-(3-trifluoromethylphenyl)-3-chloro-4-chloromethyl-2-pyrrolidinone
B: 1-methyl-3-phenyl-5-(3-trifluoromethylphenyl)pyridin-4(1H)-one

Test 2 Upland Soil Treatment Test (Pre-emergence Treatment)

Resin-made 1/2500-are pots were filled with the soil of an upland field. Corn (*Zea maize*), wheat (*Triticum*) and cotton (*Gossypium indicum*) were separately seeded. Those seeds were covered with a 2-3

cm soil in which seeds of morningglory (*Ipomoea purpurea*), foxtail (*Setaria viridis*), chickweed (*Stellaria media*), shepherdspurse (*Capsella bursapastoris*), barnyardgrass (*Echinochloa*) and crabgrass (*Digitaria adscendens*) had been mixed, and were allowed to germinate in a green house. One day later (before emergence of weeds), a wettable powder formulated from a predetermined amount of each test compound in a similar manner to the method described in Formulation Example 1 was diluted with water at predetermined dilution rates and then evenly sprayed at an application rate equivalent to 10 ℓ per are onto the surface of the soil in each pot by means of a pressure-operated ULV (ultra low volume) sprayer. Upon an elapsed time of 30 days after the spraying, influence to the crops and weeds was observed and investigated. The results are shown in Table 3, in which Comparative Compounds A and B are those described in Test 1 and the degrees of damages to the respective test plants and the degrees of injury of the crops are shown similarly to Test 1.

In the present test, the compounds according to the present invention showed, compared with Comparative Compounds A and B, higher herbicidal effects against the upland weeds tested and excellent safety to the crops tested, namely, corn (*Zea maize*), wheat (*Triticum*) and cotton (*Gossypium indicum*).

Table 3 Upland Soil Treatment Test

| Comp'd. No. | Application rate, kg/ha | Morning-glory (*Ipomoea purpurea*) | Foxtail (*Setaria viridis*) | Chickweed (*Stellaria media*) | Shepherdspurse (*Capsella bursapastoris*) | Barnyard-grass | Crabgrass (*Digitaria adscendens*) | Corn (*Zea maize*) | Wheat (*Triticum*) | Soybean (*Glycine max*) | Cotton (*Gossypium indicum*) |
|---|---|---|---|---|---|---|---|---|---|---|---|
| 1 | 0.1 | 1 | 3 | 3 | 4 | 3 | 4 | 0 | 0 | 0 | 0 |
| | 0.2 | 3 | 4 | 4 | 5 | 4 | 5 | 0 | 0 | 0 | 0 |
| | 0.4 | 4 | 5 | 5 | 5 | 5 | 5 | 0 | 1 | 0 | 1 |
| 2 | 0.1 | 2 | 4 | 3 | 4 | 3 | 4 | 0 | 0 | 0 | 0 |
| | 0.2 | 3 | 5 | 4 | 5 | 4 | 5 | 0 | 0 | 0 | 0 |
| | 0.4 | 5 | 5 | 5 | 5 | 5 | 5 | 0 | 1 | 0 | 0 |
| 3 | 0.1 | 1 | 1 | 1 | 2 | 2 | 2 | 0 | 0 | 0 | 0 |
| | 0.2 | 2 | 3 | 3 | 3 | 3 | 4 | 0 | 0 | 0 | 0 |
| | 0.4 | 5 | 4 | 4 | 4 | 5 | 4 | 0 | 0 | 0 | 0 |
| A | 0.1 | 0 | 1 | 0 | 1 | 0 | 2 | 0 | 0 | 0 | 0 |
| | 0.2 | 2 | 2 | 2 | 2 | 2 | 4 | 2 | 2 | 1 | 0 |
| | 0.4 | 3 | 4 | 4 | 4 | 4 | 5 | 2 | 2 | 2 | 2 |
| B | 0.1 | 2 | 2 | 1 | 1 | 2 | 2 | 0 | 0 | 0 | 0 |
| | 0.2 | 3 | 3 | 3 | 3 | 3 | 3 | 1 | 1 | 1 | 1 |
| | 0.4 | 4 | 4 | 4 | 4 | 5 | 4 | 2 | 2 | 3 | 2 |

Comparative Compounds:

A: 1-(3-trifluoromethylphenyl)-3-chloro-4-chloromethyl-2-pyrrolidinone

B: 1-methyl-3-phenyl-5-(3-trifluoromethylphenyl)pyridin-4(1H)-one

Test 3 Upland Foliar Application Test

Resin-made 1/10000-are pots were filled with the soil of an upland field. Morningglory (*Ipomoea purpurea*), smartweed (*Polygonum lapathifolium*), chickweed (*Stellaria media*), common lambsquarter

(*Chenopodium album*), barnyardgrass, crabgrass (*Digitaria adscendens*), corn (*Zea maize*), wheat (*Triticum*), soybean (*Glycine max*) and cotton (*Gossypium indicum*) were separately seeded and were then allowed to germinate in a green house. When each plant grew to the stage of 2-3 leaves, each emulsion formulated from a predetermined amount of each test compound in a similar manner to Formulation Example 4 was diluted with water and then sprayed at an application rate equivalent to 5 ℓ per are by means of a pressure-operated ULV (ultra low volume) sprayer. Upon an elapsed time of 30 days after the spraying of the herbicide, influence to the crops and weeds was observed and investigated. The results are shown in Table 4, in which the degrees of damages to the respective test plants and the degrees of injury of the crops are shown similarly to Test 1.

In the present test, the compounds according to the present invention showed, compared with Comparative Compounds A and B, higher herbicidal effects against the upland weeds tested and excellent safety to the crops tested, namely, corn (*Zea maize*), wheat (*Triticum*), soybean (*Glycine max*) and cotton (*Gossypium indicum*).

Table 4 Upland Foliar Application Test

| Comp'd. No. | Application rate, kg/ha | Morning-glory (*Ipomoea purpurea*) | Smartweed (*Polygonum lapathifolium*) | Chickweed (*Stellaria media*) | Common lamb-quarters | Barnyard-grass (*Echinochloa crusgalli*) | Crabgrass (*Digitaria adscendens*) | Corn (*Zea maize*) | Wheat (*Triticum*) | Cotton (*Gossypium indicum*) |
|---|---|---|---|---|---|---|---|---|---|---|
| 1 | 0.2 | 2 | 2 | 2 | 2 | 3 | 3 | 0 | 0 | 0 |
| | 0.4 | 3 | 3 | 3 | 3 | 4 | 4 | 0 | 0 | 0 |
| | 0.8 | 4 | 4 | 4 | 4 | 5 | 5 | 0 | 0 | 0 |
| 2 | 0.2 | 3 | 3 | 3 | 4 | 3 | 4 | 0 | 0 | 0 |
| | 0.4 | 4 | 4 | 4 | 5 | 4 | 5 | 0 | 0 | 0 |
| | 0.8 | 5 | 5 | 5 | 5 | 5 | 5 | 0 | 1 | 0 |
| 3 | 0.2 | 1 | 2 | 2 | 1 | 1 | 2 | 0 | 0 | 0 |
| | 0.4 | 2 | 3 | 3 | 2 | 2 | 3 | 0 | 0 | 0 |
| | 0.8 | 4 | 4 | 4 | 4 | 4 | 4 | 0 | 0 | 0 |
| A | 0.2 | 0 | 3 | 1 | 3 | 1 | 2 | 1 | 1 | 0 |
| | 0.4 | 2 | 4 | 3 | 4 | 2 | 3 | 2 | 2 | 1 |
| | 0.8 | 4 | 5 | 5 | 5 | 4 | 5 | 3 | 4 | 2 |
| B | 0.2 | 1 | 2 | 2 | 1 | 1 | 1 | 1 | 1 | 1 |
| | 0.4 | 3 | 3 | 3 | 2 | 3 | 2 | 2 | 2 | 2 |
| | 0.8 | 4 | 4 | 4 | 4 | 4 | 4 | 3 | 3 | 4 |

Comparative Compounds:
A: 1-(3-trifluoromethylphenyl)-3-chloro-4-chloromethyl-2-pyrrolidinone
B: 1-methyl-3-phenyl-5-(3-trifluoromethylphenyl)pyridin-4(1H)-one

The 4-ethyl-1-(3-trifluoromethylphenyl)-2-pyrrolidinone derivatives according to the present invention are novel compounds, and herbicidal compositions containing one or more of the compounds of the invention exhibit marked herbicidal activities even at extremely low application rates against various troublesome weeds in paddy fields or upland fields and have a broad herbicidal spectrum. Nevertheless, since they show outstanding selectivity to certain types of economic crops, especially to rice (*Oryza sativa*) in paddy

fields and soybean (*Glycine max*) and cotton (*Gossypium indicum*), they are usable with excellent safety.

**Claims**

1. A 4-ethyl-1-(3-trifluoromethylphenyl)-2-pyrrolidinone derivative represented by the following formula (I):

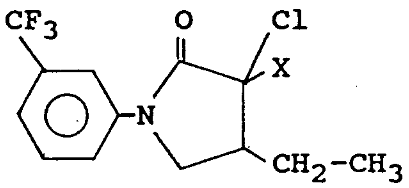

(I)

wherein X represents a chlorine or hydrogen atom.

2. The 2-pyrrolidinone derivative according to claim 1, wherein, in the formula (I), X represents a hydrogen atom and the relative positions of the chlorine atom at the 3-position and the ethyl group at the 4-position are trans.

3. A process for the preparation of a 4-ethyl-1-(3-trifluoromethylphenyl)-2-pyrrolidinone derivative represented by the following formula (I):

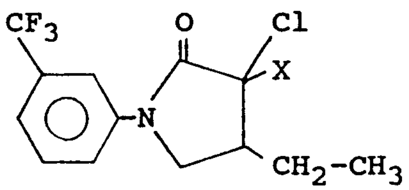

(I)

wherein X represents a chlorine or hydrogen atom, which comprises cyclizing with a trialkyltin hydride an amide derivative of the following formula (II):

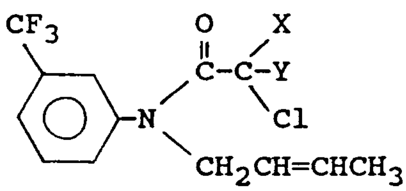

(II)

wherein X has the same meaning as defined above and Y represents a halogen atom.

4. A herbicidal composition comprising as an active ingredient a 4-ethyl-1-(3-trifluoromethylphenyl)-2-pyrrolidinone derivative represented by the following formula (I):

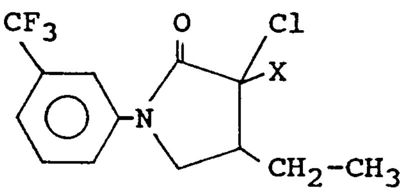

(I)

wherein X represents a chlorine or hydrogen atom.

5. The herbicidal composition according to claim 4, wherein the active ingredient is a 2-pyrrolidinone

derivative represented by the formula (I) in which X represents a hydrogen atom and the chlorine atom at the 3-position and the ethyl group at the 4-position are at positions trans to each other.

# INTERNATIONAL SEARCH REPORT

International Application No PCT/JP90/01088

**I. CLASSIFICATION OF SUBJECT MATTER** (if several classification symbols apply, indicate all) [6]

According to International Patent Classification (IPC) or to both National Classification and IPC

Int. $Cl^5$ C07D207/273, A01N43/36

**II. FIELDS SEARCHED**

Minimum Documentation Searched [7]

| Classification System | Classification Symbols |
|---|---|
| IPC | C07D207/273, A01N43/36 |

Documentation Searched other than Minimum Documentation to the Extent that such Documents are Included in the Fields Searched [8]

CAS ONLINE

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | US, A, 4909834 (ICI Americas, Inc.), 20 March 1990 (20. 03. 90) | 1 - 5 |
| Y | JP, A, 52-89666 (Stouffer Chemical Co.), 27 July 1977 (27. 07. 77), & US, A, 4069038, DE, A, 2612731 | 1 - 5 |
| Y | JP, A, 58-154558 (Stouffer Chemical Co.), 14 September 1983 (14. 09. 83), & US, A, 4110105 | 1 - 5 |
| Y | JP, A, 57-128671 (CIBA-Geigy AG), 10 August 1982 (10. 08. 82), (Family: none) | 1 - 5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| October 25, 1990 (25. 10. 90) | November 5, 1990 (05. 11. 90) |
| **International Searching Authority** | **Signature of Authorized Officer** |
| Japanese Patent Office | |